# EUROPÄISCHE PATENTSCHRIFT

(11) **EP 2 034 938 B1**
(45) Veröffentlichungstag und Bekanntmachung des Hinweises auf die Patenterteilung: **13.04.2016**
(21) Anmeldenummer: 07764922.6
(22) Anmeldetag: 28.06.2007
(51) Int. Cl.: A61F 9/01, A61F 9/008

(54) **BESTRAHLUNGSSYSTEM FÜR OPHTHALMOLOGISCHE ANWENDUNGEN**
RADIATION SYSTEM FOR OPHTHALMOLOGICAL APPLICATIONS
SYSTÈME D'IRRADIATION POUR APPLICATIONS OPHTALMOLOGIQUES

(30) Priorität: 30.06.2006 DE 102006030219
(43) Veröffentlichungstag der Anmeldung: 18.03.2009
(73) Patentinhaber: IROC Innocross AG, 6300 Zug (CH)
(72) Erfinder: MROCHEN, Michael, 8193 Eglisau (CH); BÜELER, Michael, 8048 Zürich (CH); SCHELLING, Urs, 8005 Zürich (CH)
(74) Vertreter: von Hellfeld, Axel
(86) Internationale Anmeldenummer: PCT/EP2007/005740
(87) Internationale Veröffentlichungsnummer: WO 2008/000478

(56) Entgegenhaltungen:
- EP-A1- 1 285 679
- WO-A-93/16631
- WO-A-94/03134
- WO-A-03/061696
- SOBOL E N ET AL: "CORRECTION OF EYE REFRACTION BY NONABLATIVE LASER ACTION ON THERMOMECHANICAL PROPERTIES OF CORNEA AND SCLERA" QUANTUM ELECTRONICS, TURPION LTD., LONDON, GB, Bd. 32, Nr. 10, Oktober 2002 (2002-10), Seiten 909-912, XP001170947 ISSN: 1063-7818

## Beschreibung

Die Erfindung betrifft ein Bestrahlungssystem für ophthalmologische Anwendungen zur Erzielung von Veränderungen der biomechanischen Eigenschaften von Komponenten des Auges, insbesondere der Hornhaut. Hierfür setzt die vorliegende Erfindung eine hier mit "Primärstrahlung" bezeichnete elektromagnetische Strahlung ein, bevorzugt im spektralen Bereich von 300 nm bis 800 nm. Diese Strahlung wird bevorzugt mit LEDs oder Laserdioden erzeugt. Die hier mit "Primärstrahlung" bezeichnete elektromagnetische Strahlung soll keinen sogenannten fotoablativen Effekt bewirken, also keinen Effekt, bei dem Gewebe aus dem Auge entfernt wird, wie dies bei einer Neuformung der Kornea zum Beispiel gemäß der LASIK, erfolgt. Vielmehr dient die erfindungsgemäße Primärstrahlung dazu, das Gewebe, insbesondere die Hornhaut, in ihren biomechanischen Eigenschaften zu verändern, ohne Gewebe zu entfernen. Eine Änderung der biomechanischen Eigenschaften zum Beispiel der Hornhaut liegt dann vor, wenn das Gewebe in seiner Elastizität verändert wird ("verhärtet"). Hierzu kennt der Stand der Technik sogenannte Fotosensibiliatoren, also Wirkstoffe, die in das Gewebe injiziert werden, und die dort den genannten Effekt der Änderung biomechanischer Eigenschaften des Gewebes fördern. Die erfindungsgemäße Primärstrahlung bewirkt also im Ergebnis eine biomechanische Stabilisierung der Hornhaut.

Die Druckschrift WO 93/16631 beschreibt ein Bestrahlungssystem für Ablation oder Fotokoagulation von Hornhautgewebe. Die photomechanische Stabilisierung von Hornhautgewebe, in die ein Photosensibilisator eingebracht ist, ohne photoablativen Effekt, findet jedoch mit Strahlungsintensitäten statt, die um mehrere Zehnerpotenzen verschieden sind von Strahlungsintensitäten, wie sie für eine Photoablation oder Photokoagulation eingesetzt werden.

Der Erfindung liegt die Aufgabe zugrunde, ein für die photomechanische Stabilisierung von Hornhautgewebe, ohne photoablativen Effekt, optimales Bestrahlungssystem bereitzustellen.

Hierzu dient das Bestrahlungssystem gemäß Anspruch 1.

Weitere Ausgestaltungen der Erfindung sind in den weiteren Ansprüchen und der nachfolgenden Beschreibung von Ausführungsbeispielen anhand der Figuren beschrieben.

Es zeigt:
- Figur 1: schematisch ein Bestrahlungssystem für medizinische Anwendungen;
- Figur 2: eine Einzelheit des Bestrahlungssystems;

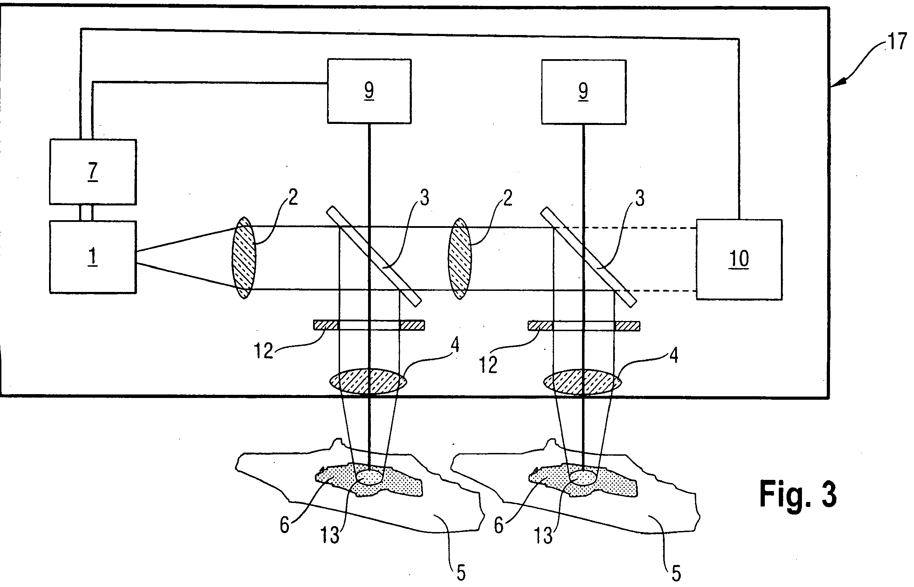

- Figur 3: eine weitere Ausführungsform eines Bestrahlungssystems für medizinische Zwecke mit zwei Subsystemen;
- Figur 4: schematisch Ausführungsbeispiele für Bestrahlungsfelder;
- Figur 5: eine weitere Einzelheit eines Bestrahlungssystems für medizinische Zwecke mit einer Kalibriereinrichtung;
- Figur 6: eine weitere Einzelheit eines Bestrahlungssystems für medizinische Zwecke;
- Figur 7: schematisch einen Adapter für ein Bestrahlungssystem der vorstehend genannten Art zur Positionierung in Bezug auf ein Auge;
- Figur 8: ein weiteres Ausführungsbeispiel für eine Adapter ähnlich Figur 7;

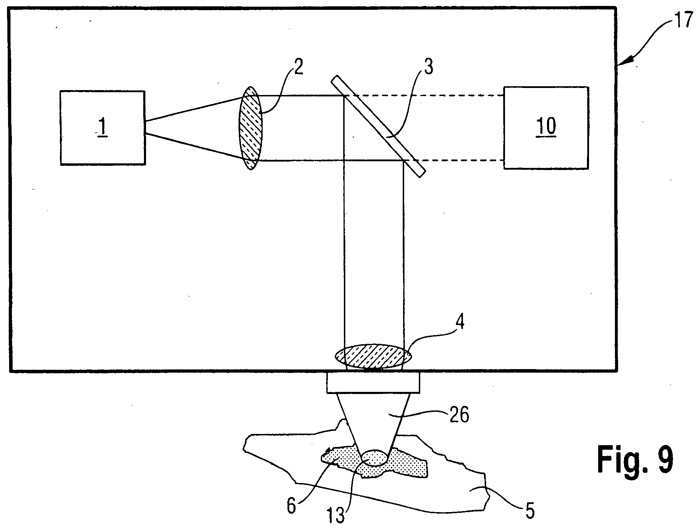

- Figur 9: das Zusammenwirken eines medizinischen Strahlungssystems mit einem Adapter;
- Figur 10: eine Zentrierung von Bestrahlungsmitteln;
- Figur 11: ein Behandlungssystem mit Einstelleinrichtungen für optische Komponenten;
- Figur 12: eine Variante der Ausführungsform von Figur 11;
- Figur 13: eine Variante von Figur 12 und
- Figur 14: eine Einrichtung zum Austausch von optischen Elementen.

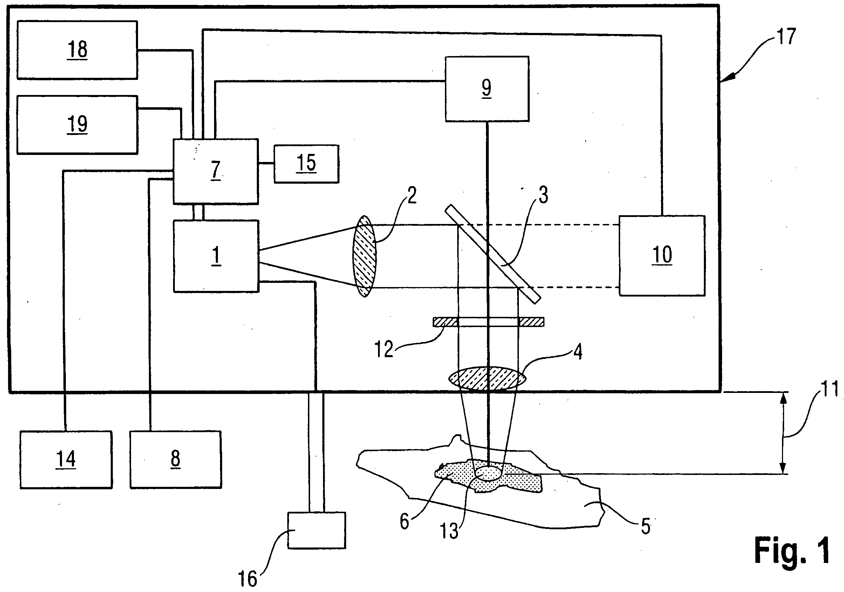

Das Bestrahlungssystem für medizinische Zwecke gemäß Figur 1 weist eine Strahlungsquelle 1 für elektromagnetische Strahlung auf, zum Beispiel eine LED, einen Laser, oder eine thermische Lichtquelle. Die Strahlung wird über eine Linse 2 und einen halbdurchlässigen Spiegel 3 sowie einer weiteren Linse 4 auf zu behandelndes Gewebe 5 fokussiert.

Die hier beschriebenen Ausführungsbeispiele der Erfindung eignen sich insbesondere für den ophthalmologischen Einsatz des Bestrahlungssystems.

In das zu behandelnde Gewebe 5 ist ein Wirkstoff eingebracht, der die fotochemischen und/oder fotophysikalischen Wirkungen der in das Gewebe eingebrachten Strahlung ermöglicht oder fördert.

Der Bestrahlungsbereich ist mit dem Bezugszeichen 13 bezeichnet.

Eine Bestrahlungseinheit 17 ist auf einem Stativ 16 so abgestützt, dass ein vorgegebener Abstand 11 zwischen dem im Strahlungsweg zuletzt angeordneten optischen Element 4 der Bestrahlungseinheit 17 und dem zu bestrahlenden Gewebe 5 einstellbar ist.

Mit dem Strahlteiler 3 können wahlweise unterschiedliche Funktionen erreicht werden:

Zum Einen kann mit dem Strahlteiler 3 ein Teil der von der Strahlungsquelle 1 emittierten Strahlung ausgekoppelt und einer Messeinrichtung 10 zugeführt werden, die zum Beispiel die Energie und/oder die Intensitätsverteilung und/oder die Zeitverteilung der Strahlung misst.

Andererseits kann mit dem Strahlteiler 3 auch ein Teil der vom Gewebe 5 zurückreflektierten Strahlung einer Beobachtungseinrichtung 9 zugeführt werden.

Gemäß einer dritten Variante kann mit dem Strahlteiler 3 die von der Strahlungsquelle 1 emittierte Strahlung mit einer weiteren Strahlung kombiniert werden, wobei die weitere Strahlung dann von einer weiteren Strahlungsquelle emittiert wird, die im mit dem Bezugszeichen 9 versehenen Bauteil angeordnet ist. Die weitere Strahlung hat dann vorzugsweise eine andere Wellenlänge als die von der Strahlungsquelle 1 emittierte Strahlung.

Eine Steuerung 7 dient zur Steuerung u.A. der genannten Komponenten 1, 10 und 9.

Die Steuerung 7 erhält Daten von einem Sensor 15, der wichtige Parameter des Bestrahlungssystems erfasst, wie zum Beispiel die Stromaufnahme der Strahlungsquelle, Temperaturen des Systems und/oder der Umgebung, die Luftfeuchtigkeit der Umgebung und weitere Größen.

Ein Netzteil 14 dient der Energieversorgung der Bestrahlungseinheit 17.

Ein Anzeigelement 18 dient zur Anzeige interessierender Parameter, wie der Lichtemission, interessierender Behandlungsparameter bezüglich des Patienten, oder auch möglicher auftretender Fehler.

Eine Schnittstelle 8 ist mit der Steuerung 7 verbunden und dient dem Anschluss derselben an einen externen Rechner (nicht gezeigt), zum Übertragen von behandlungsrelevanten Daten, wie zum Beispiel den Bestrahlungsdauern, der Bestrahlungsdosis, der Lichtverteilung, interessierenden Messdaten, oder auch zur Übertragung von Daten aus einer Datenbank.

Eine Schnittstelle 19 kann vorgesehen sein zum Übermitteln von Daten an die Steuerung 7 und insbesondere zum Anschluss eines PC mit Eingabeeinrichtungen für den Benutzer bezüglich der Einzelheiten der Behandlung.

In den Figuren sind einander entsprechende oder funktionsähnliche Bauteile mit den gleichen Bezugszeichen versehen.

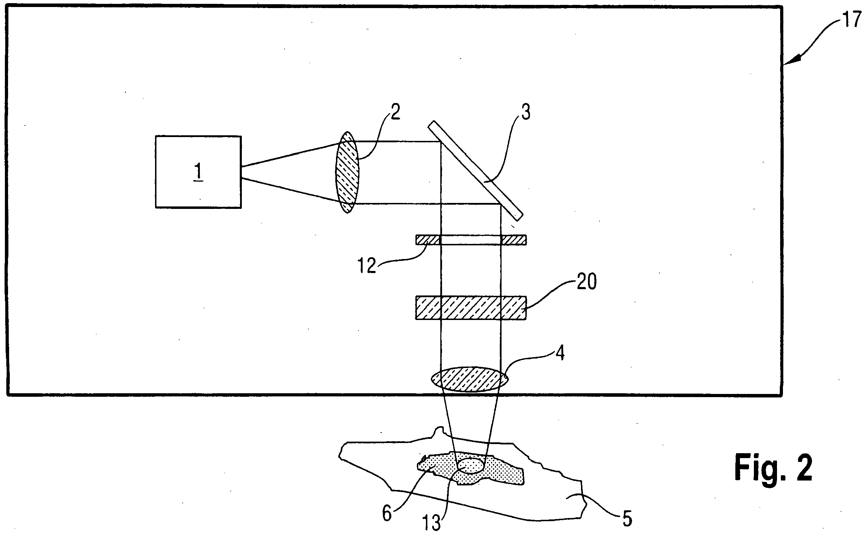

Figur 2 zeigt eine Einzelheit des in Figur 1 dargestellten Bestrahlungssystems, wobei in der Behandlungseinheit 17 im Strahlengang vor der Linse 4 ein optisches Element 20 angeordnet ist, das eine diffraktive oder holografische Wirkung hat, um im Bestrahlungsbereich 13 eine vorgegebene und auswählbare Lichtverteilung zu erzeugen.

In Abwandlung des vorstehend beschriebenen Ausführungsbeispieles kann das optische Element 20 auch ein zeitlich veränderbarer Lichtmodulator sein, zum Beispiel ein Flüssigkristallmodulator, um im Bestrahlungsbereich 13 eine auswählbare Lichtverteilung zu erzeugen.

In Abwandlung der vorstehend beschriebenen Ausführungsbeispiele kann das optische Element 20 auch durch Bewegung im Raum eine einstellbare und veränderliche Lichtverteilung im Gewebe 5 erzeugen.

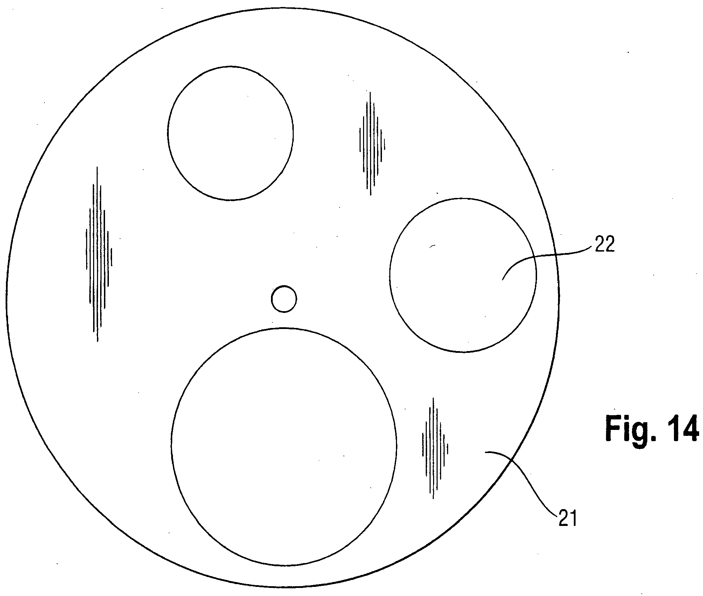

Gemäß einer weiteren Variante kann vorgesehen sein, an der Stelle des optischen Elementes 20 ein in Figur 14 gezeigtes Wechselrad (Revolver) 21 anzuordnen, mit dem unterschiedliche optische Elemente, wie zum Beispiel Absorber 22 in den Strahlungsweg bewegbar sind, um eine vorgegebene Lichtverteilung im Strahlungsbereich 13 zu erzeugen. Dabei ist in den zu bestrahlenden Bereich des Gewebes 5 ein chemische Wirkstoff 6 (Figur 2) eingebracht, um den physikalischen oder chemischen Effekt der Strahlung zu ermöglichen oder zumindest zu fördern. Solche chemischen Wirkstoffe sind als solche bekannt.

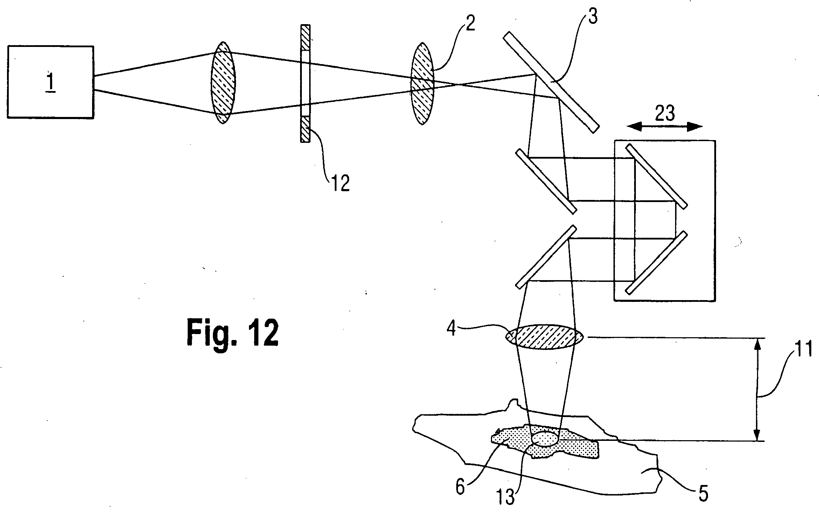

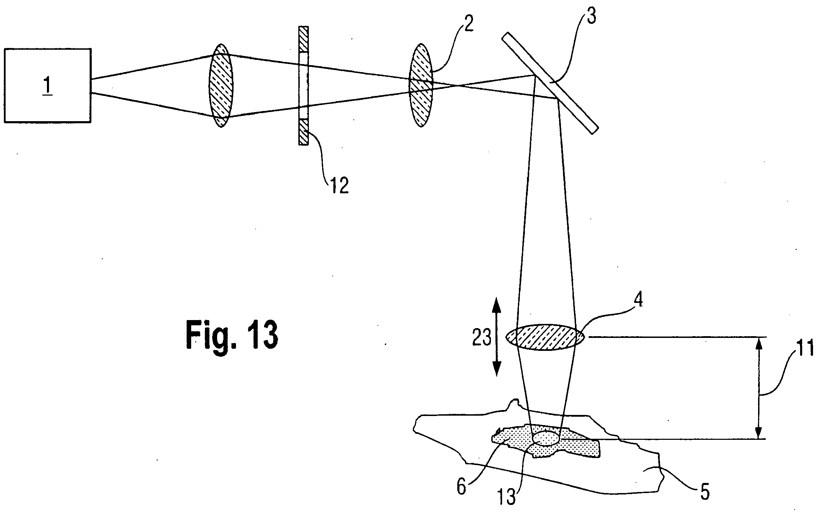

Die Figuren 12 und 13 zeigen Abwandlungen der vorstehend beschriebenen Ausführungsbeispiele, wobei optische Elemente, wie durch den Pfeil 23 angedeutet, im Raum verfahrbar sind, um die Lichtverteilung im Bestrahlungsfeld 13 einzustellen.

Diese Ausführungsbeispiele ermöglichen auch, dass der Abstand 11 zwischen der Bestrahlungseinheit 17 und dem zu bestrahlenden Gewebe 5 einstellbar ist.

Die in Figur 1 gezeigte Blende 12 ist vorzugsweise in ihren Blendenöffnung und/oder hinsichtlich ihres Abstandes in Bezug auf die anderen optischen Elemente steuerbar (einstellbar).

Gemäß einer Variante kann die Blende 12 als eine rotierende Maske ausgestaltet sein derart, dass durch die rotierende Blende (Maske) jeweils bei der Rotation unterschiedliche Bereiche des Strahls ausgeblendet werden, sodass es zu einer zeitlich und räumlich variierenden Strahlungsdosis auf dem Gewebe 5 kommt. So erzeugt zum Beispiel eine spiralförmige Maske eine parabolische Lichtverteilung im Strahlungsbereich 13.

Nachfolgend wird das Bestrahlungssystem hinsichtlich des im Block 9 wahlweise vorgesehenen Diagnosemittels näher beschrieben:

Das Messmittel 9 kann zum Beispiel ein Gerät für die optische Kohärenztomographie sein. Wahlweise kann das Messmittel 9 auch ein Gerät zur Messung der optische Augenlänge oder ein Messmittel zur Ermittlung der Hornhauttopographie in Echtzeit sein.

Eine andere Ausgestaltung sieht vor, dass das Messmittel 9 ein Wellenfrontdiagnosesystem ist zur Messung der Wellenfront in Echtzeit, die vom Gewebe 5 zurückgestrahlt wird.

Wahlweise kann das Messmittel 9 auch eine Scheimpflugkamera sein.

Eine andere Ausgestaltung sieht vor, dass das Messmittel 9 ein Videosystem zur Bildgabe ist. Auch kann das Messmittel 9 ein Kamerasystems zur elektronischen Bildgabe sein.

Eine andere Ausgestaltung sieht vor, dass das Messmittel 9 ein Mikroskop ist zur visuellen Beobachtung der Behandlung.

Andererseits kann das Messmittel 9 ein Spektrometer zur Fluoreszenzanalyse sein.

Wiederum in einer anderen Ausgestaltung kann es sich bei dem Messmittel 9 um ein System zur Erfassung der Augenbewegungen (sogenannter eye tracker) sein.

Eine andere Ausgestaltung sieht vor, dass das Messmittel 9 ein Gerät ist zur Messung der Hornhaut und/oder der Epitheldicke.

Auch kann es sich bei dem Messmittel 9 um ein Gerät zur Abstandsmessung vom Auge (bezogen auf die optischen Komponenten) handeln.

Nachfolgend werden unterschiedliche Funktionen des Stahlteilers 3 gemäß unterschiedlichen Ausführungsbeispielen der Erfindung erläutert:

Wie oben bereits angedeutet, dient der Strahlteiler 3 bei einer ersten Variante der Erfindung dazu, mit der von der Strahlungsquelle 1 kommenden Strahlung (sogenannte Primärstrahlung) eine Strahlung einer anderen Wellenlänge zu kombinieren, wobei die Strahlungsquelle für die zweite Strahlung (Sekundärstrahlung) im mit 9 bezeichneten Block sitzt. Bei der Sekundärstrahlung kann es sich zum Beispiel um Strahlung mit einer Wellenlänge handeln, die zur UV-Fotoablation von Hornhaut geeignet ist.

Gemäß einer anderen Variante der Erfindung kann die Sekundärstrahlung so ausgewählt sein, dass sie im bestrahlten Gewebe eine Fluoreszenzwirkung erzielt, wobei dann Einrichtungen vorgesehen sind, um die Fluoreszenzstrahlung auszuwerten.

Eine weitere Variante des Einsatzes des Strahlteilers 3 sieht vor, dass die Sekundärstrahlung eine Wellenlänge hat, die geeignet ist, um das zu bestrahlende Gewebe 5 thermisch anzuregen und so die gewünschten Effekte zu fördern.

Eine weitere Variante der Funktion des Strahlteilers 3 ist eine Sekundärstrahlung, die so ausgewählt ist, dass sie im sichtbaren optischen Bereich (für den Patienten sichtbar) liegt, und als sogenannter Fixationsstrahl oder Zielstrahl dient.

Gemäß einer anderen Variante dient der Strahlteiler 3 dazu, die Primärstrahlung in zwei Strahlengänge aufzuteilen, sodass ein (kleinerer) Strahlteil zu Messzwecken in eine Messeinrichtung 10 eingegeben werden kann. Dabei wird das Signal der Messeinrichtung 10 in die Steuerung 7 zur Verarbeitung gegeben.

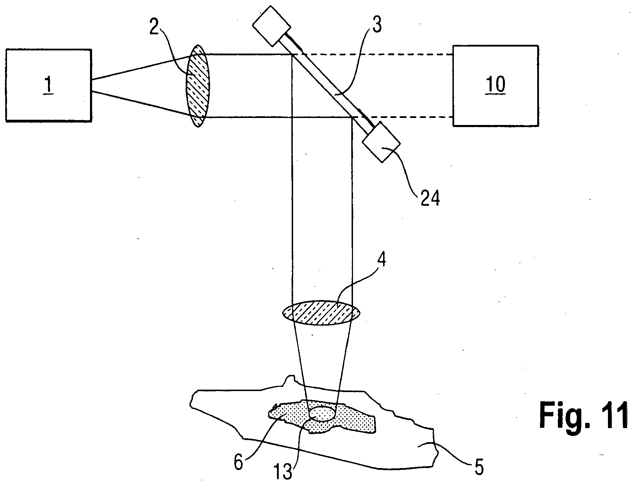

Gemäß einer anderen Variante ist der Strahlteiler 3 durch elektrisch ansteuerbare Mittel 24, siehe Figur 11, so bewegbar, dass der Bestrahlungsbereich 13 über das Gewebe 5 geführt ("gescanned") werden kann.

Nachfolgend werden Einzelheiten der Steuerung 7 erläutert:

Die Steuerung 7 kann so ausgelegt sein, dass sie die genannte Primärstrahlung zeitlich pulsierend abgibt oder kontinuierlich.

Auch kann die Steuerung 7 so programmiert sein, dass die Leistung der primären Strahlung zeitlich variierend einstellbar ist. Dabei sieht eine besondere Ausgestaltung vor, dass die Leistung der von der Quelle 1 abgegebenen Primärstrahlung vor einem eigentlichen Behandlungsbeginn über eine vorgegebene Zeitspanne unterhalb einem vorgegebenen Schwellenwert gehalten wird, um innerhalb dieser vorgegebenen Zeitspanne Justierungen oder Messungen mit der Strahlung durchzuführen. Nach der Zeitspanne kann dann die Strahlung über den genannten Schwellenwert angehoben werden, um eine erwünschte chemische und/oder physikalische Wirkung zu erzielen.

Die Steuerung 7 kann über einen Fusstaster zur Aussendung der Strahlung steuerbar sein. Es ist auch möglich, die Steuerung 7 über eine Fernbedienung zur Aussendung der Strahlung zu bedienen.

Sind mehrere Strahlungsquellen, zum Beispiel mehrere LEDs zur Erzeugung der Primärstrahlung vorgesehen, kann die Steuerung 7 einzelne der Strahlungsquellen jeweils ansteuern, um einen gewünschten räumlichen und/oder zeitlichen Intensitätsverlauf der Strahlung zu steuern.

Der insbesondere eine Messeinrichtung anzeigende Block 10 gemäß Figur 1 ist insbesondere ein Fotodetektor, mit dem die Strahlungsdosis pro Zeiteinheit und über den zeitlichen Verlauf der Behandlung gemessen wird. Dabei kann vorgesehen sein, dass von der Messeinrichtung 10 ein Signal an die Steuerung 7 gegeben wird, um den zeitlichen Verlauf der Strahlung in der Behandlungsebene gemäß einem vorgegebenen Programm zu steuern. Treten Abweichungen hinsichtlich eines gemessenen Parameters gegenüber dem Soll-Verlauf des Programms auf, kann in der Art eines geschlossenen Regelkreises die Steuerung 7 die Strahlung so ändern, dass der genannte Parameter wieder im Soll liegt.

Bezeichnet der Block 9 in Figur 1 einen sogenannten Eye-Tracker, dann kann ein entsprechendes Signal über die Bewegung des Auges an die Steuerung 7 gegeben werden (in den Figuren zeigen die Verbindungslinien zwischen den Funktionsblöcken den wechselseitigen Datenaustausch an) und die Steuerung 7 kann dann einen Motor 24 (Figur 11) betätigen, um den beweglichen Strahlteiler 3 entsprechend der Bewegung des Auges nachzuführen.

Gemäß einer weiteren Ausgestaltung ist die Steuerung 7 so ausgelegt, dass sie das bewegbare Stativ 16 ansteuert und so die Position der Bestrahlungseinheit 17 in Bezug auf das Gewebe 5 einstellt.

Die Steuerung 7 ist so programmiert, dass sie über eine Schnittstelle von z.B. einem Rechner erhaltene Daten, insbesondere bezüglich der Hornhautdicke, der Epitheldicke, der Riboflavinkonzentration (letzteres ist ein Beispiel für eine Wirksubstanz 6 im Gewebe 5) verrechnet, um für die Behandlung optimale Werte hinsichtlich der Dosis und des zeitlichen Verlaufs der Intensität zu ermitteln und dann entsprechend das System zu steuern.

In ähnlicher Weise kann die Steuerung 7 auch über eine Schnittstelle erhaltene Messdaten hinsichtlich optischer Parameter, also insbesondere hinsichtlich der Wellenfronten und der Topographie, auswerten, um optimale Behandlungsdaten zu ermitteln und das System entsprechend zu steuern.

Auch können analog präoperative und postoperative Messdaten verwendet werden, um für die Behandlung optimale Strahlungsparameter zu berechnen.

Bevorzugt erfolgt die Berechnung von Daten in der Steuerung 7 in Echtzeit (online).

Nachfolgend werden einige Ausgestaltungen des Stativs erläutert:

Das Stativ 16 (Figur 1) dient allgemein zur Positionierung der Bestrahlungseinheit 17 in Bezug auf das zu bestrahlende Gewebe. Zum Beispiel kann es sich um ein Tischstativ handeln. Hierzu kann das Stativ einen Federgelenkarm aufweisen, also einen Arm, der über Federn in einer Ausgangslage als Ruhestellung vorgespannt ist und der aus dieser Ausgangslage durch einen Benutzer verschwenkbar ist und in der verschwenkten Lage dann arretierbar ist. Es ist auch möglich, das mechanische Stativ 16 so zu gestalten, dass es durch Elektromotoren positionierbar ist, ein-dimensional, zwei-dimensional oder drei-dimensional.

Es ist auch möglich, das Stativ 16 direkt mit einem Patientenbett oder Patientenstuhl zu verbinden.

Nachfolgend werden einige Eigenschaften der Benutzerschnittstelle 19 erläutert:

Die Benutzerschnittstelle 19 ermöglicht insbesondere die Eingabe des zeitlichen und räumlichen Verlaufs der Strahlungsintensitäten. Dabei ist insbesondere ein über die Zeit veränderlicher Verlauf der Intensitätsverteilung vorgesehen.

Weiterhin ermöglicht die Benutzerschnittstelle 19 die Eingabe von Patientendaten, wie Hornhautdicke, Epitheldicke, Konzentration und Art des Wirkstoffes 6 im Gewebe, wobei letzterer auch als "Photosensitizer" bezeichnet wird. Auch können über die Benutzerschnittstelle 19 optische Messdaten eingegeben werden.

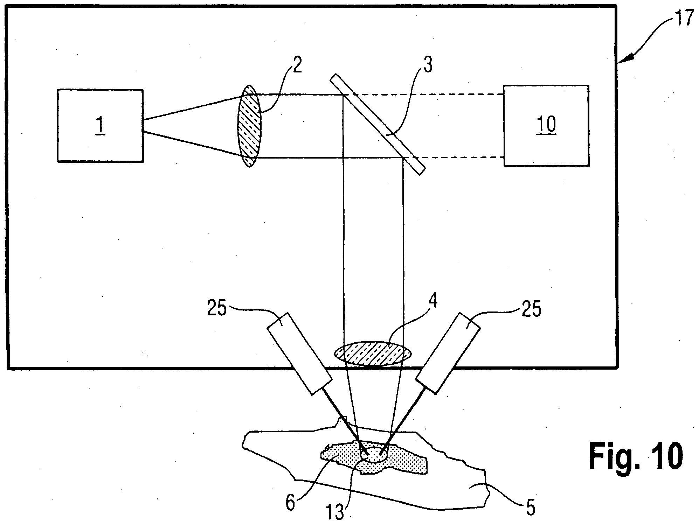

Nachfolgend werden Ausführungsformen gemäß Figur 10 erläutert:

Figur 10 zeigt zwei Lichtquellen 25, die insbesondere als Laserdioden ausgebildet sein können. Diese Strahlen sind ebenfalls auf den Bestrahlungsbereich 13 gerichtet (Figur 10). Sie dienen der räumlichen Justierung und insbesondere Zentrierung des Systems. Die Strahlung der Lichtquellen 25, die vom Gewebe 5 reflektiert wird, kann zum Beispiel über die Linse und den teildurchlässigen Spiegel 3 aufgrund der Wellenlängen separiert werden und mit einem Kamerasystem (an der Stelle des Blockes 9) ausgewertet werden, um eine räumliche Justierung der Strahlung zu ermöglichen. Dafür sind insbesondere der über die Linse 4 auf das Gewebe 5 gerichtete Strahl und der Justierstrahl zumindest einer der Lichtquellen 25 im Sollzustand kozentrisch. Der Winkel, unter dem der Strahl der zumindest einen Lichtquelle 25 (in Figur 10 sind zwei gezeigt) auf das Gewebe 5 auftrifft, ist vorgegeben und genau bekannt.

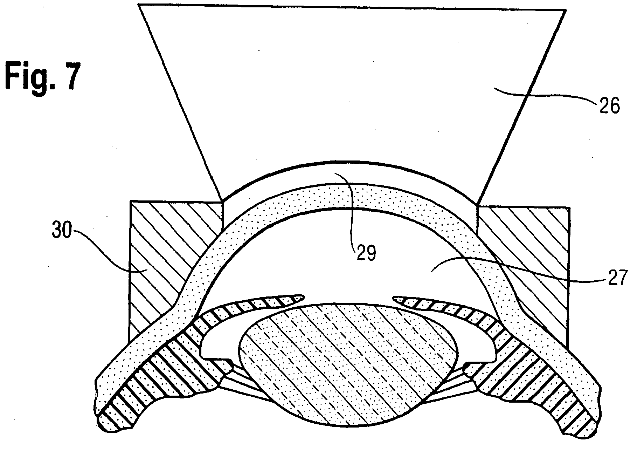

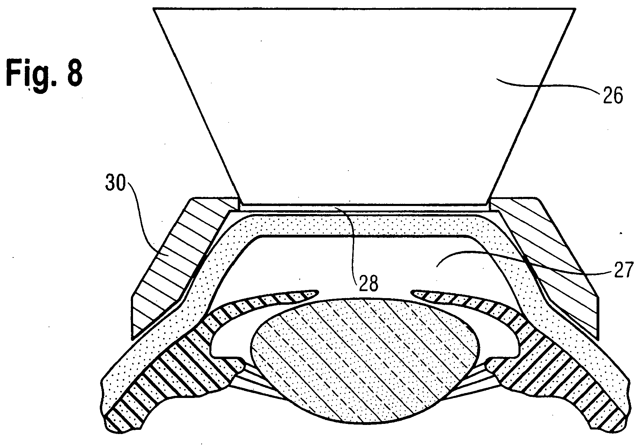

Nachfolgend werden mit Bezugnahme auf die Figuren 7 und 8 einige Einzelheiten einer Einrichtung zum Positionieren des Bestrahlungssystems in Bezug auf ein zu behandelndes Auge erläutert:

Die Bestrahlungseinheit 17 ist über einen Adapter 26 (Figur 7) in Bezug auf das Auge positioniert. Die Figuren 7 und 8 zeigen schematisch die Bestandteile des Auges 27. Der Adapter 26 hat insgesamt eine gesichtsförmige Schale, sodass Kopfbewegungen während der Bestrahlung verhindert sind.

Der Adapter 26 hat weiterhin eine Applanationsform 29, 28 (vgl. Figur 7, Figur 8), die transparent für die verwendete und gegebenenfalls die reflektierte Strahlung ist. Die Applanationsform 28 wird auf die Hornhaut gedrückt und verformt die Hornhaut in gewünschter Weise. Zum Beispiel kann die Form der Applanationsform 28 entsprechend der Diagnose sphärisch, asphärisch bitorisch, oder durch ein Zernike-Polynom beschrieben sein. Das Zernike-Polynom kann bis zur 10. Ordnung gehen.

Der Applikator 26, der die Kornea allseitig umfasst und stützt, kann gemäß einer Ausgestaltung mit Mitteln versehen sein, um das in das Gewebe zu injizierende Medikament abzugeben, und zwar in bestimmten Dosen. Es kann im Applikator eine kleine Pumpe vorgesehen sein, die elektrisch durch die Steuerung 7 ansteuerbar ist, um das Medikament in die Hornhaut zu überführen.

Die Applanationsform des Adapters 26, also die Form, mit dem der Adapter das zu behandelnde Gewebe, also insbesondere die Hornhaut, durch sanftes Andrücken formt, kann so ausgestaltet sein, dass das Gewebe nur in Teilen, also bestimmten ausgewählten Bereichen, geformt wird. Diese geformten Bereiche können innerhalb und/oder außerhalb der bestrahlten Zone liegen.

Der mechanische Adapter 26 kann Sensoren aufweisen, deren Anordnung durch das Bezugszeichen 30 angedeutet ist. Zum Beispiel können die Sensoren biomechanische Eigenschaften des Gewebes ermitteln. Auch könnend die Sensoren 30 vorgesehen sein, um die Konzentration des chemischen Wirkstoffes im Gewebe zu ermitteln.

Auch können die Sensoren 30 ausgebildet sein, um eine Wirkstoffkonzentration in der Vorderkammer des Auges zu ermitteln.

Insgesamt kann der Adapter 26 mit einer mechanischen Ansaugvorrichtung an das Auge 27 versehen sein. Dabei kann ein Sensor vorgesehen sein, um die Aufpresskraft an das Auge zu messen und an die Steuerung 7 ein entsprechendes Signal zu geben.

Auch kann der Adapter 26 mit einem mechanischen System versehen sein, um das Gewebeepithel zu entfernen.

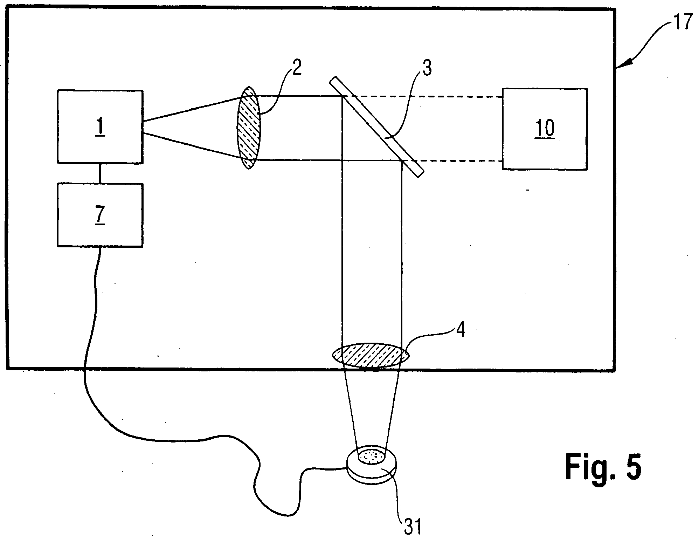

Nachfolgend wird insbesondere mit Blick auf Figur 5 ein externes Kalibriersystem beschrieben:

In der Anordnung gemäß Figur 5 wird eine Bestrahlungseinheit 17 in ihren hier interessierenden Komponenten (ansonsten entspricht sie Figur 1) dargestellt, ohne Zusammenwirken mit einem zu behandelnden Auge. Das Auge ist ersetzt durch ein Kalibriermittel 31. Mit dem Kalibriersystem 31 wird die Funktion des Bestrahlungssystems geprüft, bevor sie am Auge zum Einsatz kommt.

Bei dem Kalibriermittel 31 kann es sich zum Beispiel um einen Energiesensor, ein Spektrometer, eine Strahlprofilkamera, eine Zeitmesseinrichtung, ein Fotometer, oder ein Fluoreszenzmedium für die Wirkungsstrahlung handeln.

Das Kalibriermittel 31 liefert Signale an die Steuerung 7, sodass über die Steuerung ein geschlossener Regelkreis bezüglich der über die Strahlungsquelle 1 abgegebenen Strahlung erzeugbar ist.

Das Kalibriermittel 31 kann auch in den Applikator integriert sein und dann während der Behandlung eingesetzt werden.

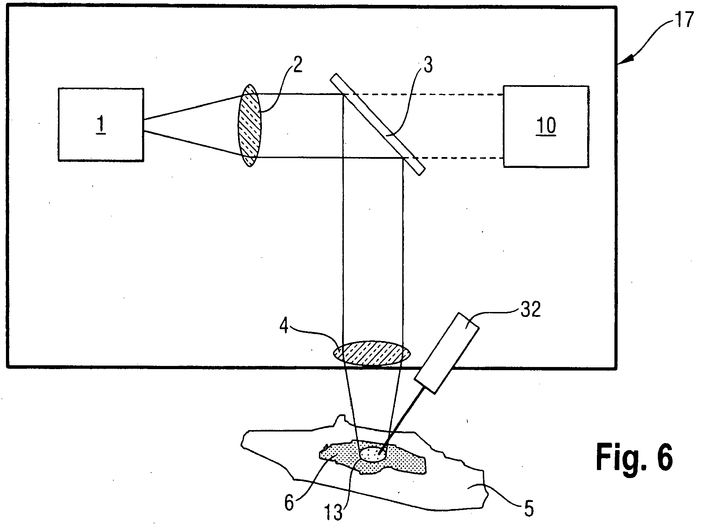

Nachfolgend wird mit Blick auf Figur 6 ein Applikator 32 für Medikamente beschrieben:

Gemäß Figur 6 ist ein Applikator 32 für Medikamente nahe der Bestrahlungsfläche 13 am oder im zu behandelnden Gewebe angeordnet. Bei dem Applikator 32 kann es sich um eine Injektor, ein Tropfsystem, oder ein Spraysystem handeln. Auch kann der Applikator 32 über die Steuerung in seiner Abgabe von Medikamenten gesteuert werden.

Die Steuerung des Applikators 32 über die Steuerung 7 kann bevorzugt in Kombination mit einer Diagnostik während der Behandlung mittels des im Block 9 angeordneten Diagnosemittels erfolgen, also in Form eines geschlossenen Regelkreises.

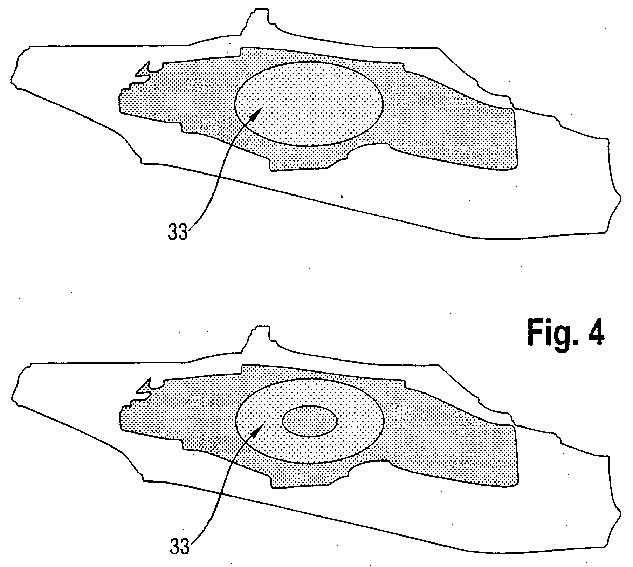

Figur 4 zeigt besondere Ausgestaltungen der Bestrahlung des Gewebes. Der Bestrahlungsbereich 13 kann danach zum Beispiel die in Figur 4 gezeigten besonderen Ausgestaltungen 33 aufweisen, also zum Beispiel eine geschlossene Kreisform gemäß Figur 4, oben, oder ein Kreisringform gemäß Figur 4, unten. Für das Bestrahlungsfeld können auch eine Ellipsenform mit bestimmter Exzentrizität gewählt werden. Die genannten Lichtformen können auch kombiniert werden, zum Beispiel in zeitlicher Abfolge, je nach der Diagnose.

## Patentansprüche

1. Bestrahlungssystem für ophthalmologische Anwendungen mit Bestrahlung von Gewebe (5), folgende Komponenten aufweisend:
a. zumindest eine Strahlungsquelle (1) für eine Primärstrahlung im Bereich von 300 nm bis 800 nm,
b. ein optisches System mit zumindest zwei Linsen (2, 4) und Einrichtungen, um die Strahlung in einem vorgegebenen Abstand auf das zu bestrahlende Gewebe zu richten, wobei Mittel (7) vorgesehen sind, um eine zeitlich und/oder räumlich veränderbare Intensitätsverteilung der Strahlung einzustellen,
c. zumindest eine Blende (12), die so ausgelegt und angeordnet ist, dass sie zusammen mit dem optischen System einen vorgegebenen Bestrahlungsbereich (13) erzeugt,
d. zumindest einen Strahlteiler (3), der einen Teil der Strahlung zu Mess- oder Überwachungszwecken auskoppelt und/oder zu Beobachtungszwecken und/oder für eine Echtzeit-Diagnostik und/oder für eine Zusammenführung der genannten Primärstrahlung mit einer weiteren Strahlung anderer Wellenlänge aus einer weiteren Strahlungsquelle eingerichtet ist,
e. eine Steuerung (7) zum Steuern oder Regeln zumindest einer der Strahlungsquellen unter Einschluss zumindest eines Sensors,
f. ein elektrisches Netzteil zur Energieversorgung des Bestrahlungssystems,
g. ein mechanisches Stativ (16) zur Abstützung einer Bestrahlungseinheit (17), welche zumindest die in den Merkmalen a., b., c., d., genannten Komponenten enthält, in Bezug auf das zu bestrahlende Gewebe (5),
h. eine Anzeigeeinrichtung (18) zum Anzeigen von für das Bestrahlungssystem relevanten Daten, wie der Strahlungsemission, Behandlungsparametern oder möglichen Fehleinstellungen,
i. eine elektronische Schnittstelle (8) zwischen der Steuerung (7) und einem externen Rechner zur Übertragung von Daten, wie zum Beispiel Bestrahlungsdauer, Dosis, Lichtverteilung, Messdaten, Daten aus Datenbänken,
j. eine Schnittstelle (19) oder eine Eingabeeinrichtung zur Eingabe von Daten durch einen Benutzer, wie zum Beispiel Bestrahlungszeiten, Bestrahlungsdosis,
**dadurch gekennzeichnet, dass**
k. das Bestrahlungssystem eingerichtet ist zur biomechanischen Stabilisierung der Hornhaut eines Auges, in die ein Photosensibilisator, wie Riboflavin, eingebracht ist,
l. wobei sich die in die Schnittstelle (19) eingegebenen Daten auf biomechanische Stabilisierung der Hornhaut mit dem Photosensibilisator, wie Riboflavin, beziehen, und
m. wobei die Steuerung (7) dazu eingerichtet ist, die zumindest eine Strahlungsquelle (1) zu steuern oder zu regeln, um Strahlung gemäß der eingegebenen Daten in Bezug auf biomechanische Stabilisierung der Hornhaut mit dem Photosensibilisator bereitzustellen,
n. wobei die zumindest eine Blende (12) steuerbar ist, um den Bestrahlungsbereich (13) anzupassen.

2. Bestrahlungssystem nach Anspruch 1 mit zumindest einem diffraktiven oder holographischen Element (20) im Strahlengang.

3. Bestrahlungssystem nach Anspruch 1 mit mindestens einem Lichtmodulator im Strahlengang zum Erzeugen einer vorgegebenen Lichtverteilung.

4. Bestrahlungssystem nach Anspruch 1 mit verfahrbaren optischen Elementen (23) im Strahlengang.

5. Bestrahlungssystem nach Anspruch 1, wobei die Blende (12) elektronisch steuerbar ist und sich im Strahlengang befindet.

6. Bestrahlungssystem nach Anspruch 1 mit Messmitteln (9) zur Echtzeitdiagnostik, insbesondere für die optische Kohärenztomographie oder zur optischen Messung der Augenlänge.

7. Bestrahlungssystem nach Anspruch 1 mit zumindest einem der folgenden Messmittel: Hornhauttopographie, Wellenfrontdiagnose, Scheimpflugkamera, Videosystem, Kamerasystem, Mikroskop, Spektrometer, Eye-Tracker, Pachymeter, oder Messung der Epitheldicke, Abstandsmessung zum Auge.

8. Bestrahlungssystem nach Anspruch 1 mit einem Strahlteiler (3) zur Zusammenfügung von Primärstrahlung aus einer ersten Strahlungsquelle (1) mit Sekundärstrahlung, die eine andere Wellenlänge hat als die Primärstrahlung, wobei es sich bei der Sekundärstrahlung insbesondere um UV-Strahlung zur Fotoablation, Strahlung zur Anregung einer Fluoreszenz des Gewebes, Strahlung für die thermische Anregung des Gewebes, oder einen Ziel- oder Fixationsstrahl handelt.

9. Bestrahlungssystem nach einem der vorhergehenden Ansprüche mit Mitteln (25) zum Justieren und Zentrieren der Strahlung.

10. Bestrahlungssystem nach einem der vorhergehenden Ansprüche, mit einem Adapter (26) zum Ausrichten des Bestrahlungssystems und Formen des zu bestrahlenden Gewebes.

11. Behandlungssystem nach einem der vorhergehenden Ansprüche, mit einem externen Kalibriersystem (31).

12. Bestrahlungssystem nach einem der vorhergehenden Ansprüche, wobei die Steuerung (7) dazu eingerichtet ist, die zumindest eine Strahlungsquelle (1) zeitlich zu steuern oder zu regulieren, gemäß einer Eingabe einer Strahlungsdosis über die Schnittstelle (19).

## Claims

1. Irradiation system for ophthalmological applications with irradiation of tissue (5), comprising the following components:
a. at least one radiation source (1) for a primary radiation within the range from 300 nm to 800 nm,
b. an optical system with at least two lenses (2, 4) and devices, in order to direct the radiation at a predetermined distance towards the tissue to be irradiated, wherein means (7) are provided in order to adjust a temporally and/or spatially variable intensity distribution of the radiation,
c. at least one diaphragm (12) which is designed and arranged in such a way that together with the optical system it generates a predetermined irradiation region (13),
d. at least one beam-splitter (3) which couples out a part of the radiation for measuring or monitoring purposes and/or is configured for observation purposes and/or for a real-time diagnosis and/or for bringing said primary radiation together with a further radiation of a different wavelength from a further radiation source,
e. a controller (7) for controlling or regulating at least one of the radiation sources, including at least one sensor,
f. an electrical power pack for supplying power to the irradiation system,
g. a mechanical stand (16) for supporting an irradiation unit (17) which includes at least the components stated in features a., b., c., d., in relation to the tissue (5) to be irradiated,
h. a display device (18) for displaying data that are relevant for the irradiation system, such as the emission of radiation, treatment parameters or possible misadjustments,
i. an electronic interface (8) between the controller (7) and an external computer for the purpose of transmitting data such as, for example, duration of irradiation, dose, light distribution, measured data, data from databases,
j. an interface (19) or an input device for the input of data by a user, such as, for example, irradiation times, irradiation dose,
**characterized in that**
k. the irradiation system is configured for biomechanical stabilisation of the cornea of an eye, into which a photosensitiser, such as riboflavin, has been introduced,
l. wherein the data input into the interface (19) relate to biomechanical stabilisation of the cornea with the photosensitizer, such as riboflavin, and
m. wherein the controller (7) is configured to control or to regulate the at least one radiation source (1), for providing radiation according to the input data in relation to biomechanical stabilisation of the cornea with the photosensitiser,
n. wherein the at least one diaphragm (12) is controllable in order to adjust the irradiation region (13).

2. Irradiation system according to claim 1, with at least one diffractive or holographic element (20) in the beam path.

3. Irradiation system according to claim 1, with at least one light modulator in the beam path for the purpose of generating a predetermined light distribution.

4. Irradiation system according to claim 1, with displaceable optical elements (23) in the beam path.

5. Irradiation system according to claim 1, wherein the diaphragm (12) is electronically controllable and is located in the beam path.

6. Irradiation system according to claim 1, with measuring means (9) for realtime diagnosis, in particular for optical coherence tomography or for optical measurement of the length of the eye.

7. Irradiation system according to claim 1, with at least one of the following measuring means: cornea topography, wavefront diagnosis, Scheimpflug camera, video system, camera system, microscope, spectrometer, eye tracker, pachymeter, or measurement of the thickness of the epithelium, distance measurement to the eye.

8. Irradiation system according to claim 1, with a beam-splitter (3) for joining primary radiation from a first radiation source (1) together with secondary radiation, which has a different wavelength from that of the primary radiation, wherein the secondary radiation is, in particular, UV radiation for photoablation, radiation for exciting a fluorescence of the tissue, radiation for the thermal excitation of the tissue, or a target beam or fixation beam.

9. Irradiation system according to any one of the preceding claims, with means (25) for adjusting and centring the radiation.

10. Irradiation system according to any one of the preceding claims, with an adapter (26) for aligning the irradiation system and shaping the tissue to be irradiated.

11. Treatment system according to any one of the preceding claims, with an external calibration system (31).

12. Treatment system according to any one of the preceding claims, wherein the controller (7) is configured to temporally control or regulate the at least one radiation source (1), according to an input of a radiation dose via the interface (19).

## Revendications

1. Système d'irradiation pour applications ophtalmologiques, destiné à l'irradiation de tissus (5) et comprenant les composants suivants :
a. au moins une source de rayonnement (1) pour générer un rayonnement primaire émis dans la plage comprise entre 300 nm et 800 nm,
b. un système optique comportant au moins deux lentilles (2, 4) et des dispositifs pour diriger le rayonnement sur le tissu à irradier depuis une distance prédéterminée, des moyens (7) étant prévus pour régler dans le temps et/ou dans l'espace une répartition variable de l'intensité du rayonnement,
c. au moins un obturateur (12) conçu et disposé de manière à générer conjointement avec ledit système optique une zone d'irradiation (13) prédéterminée,
d. au moins un diviseur de faisceau (3) qui découple une partie du rayonnement à des fins de mesure ou de surveillance et/ou qui est conçu à des fins d'observation et/ou de diagnostic en temps réel et/ou pour combiner ledit rayonnement primaire avec un autre rayonnement d'une autre longueur d'onde émis par une autre source de rayonnement,
e. une commande (7) pour commander ou régler au moins une des sources de rayonnement, laquelle commande inclue au moins un détecteur,
f. un bloc d'alimentation électrique pour assurer l'alimentation en énergie du système d'irradiation,
g. un pied mécanique (16) pour supporter une unité d'irradiation (17), laquelle comporte au moins les composants désignés dans les caractéristiques a., b., c., d., par rapport au tissu (5) à irradier,
h. un dispositif de visualisation (18) pour faire s'afficher les données pertinentes pour le système d'irradiation, telles que l'émission de rayonnement, les paramètres de traitement ou les erreurs de réglage possibles,
i. une interface électronique (8) entre la commande (7) et un calculateur externe pour la transmission de données, telles que la durée d'irradiation, la dose, la répartition de la lumière, les données mesurées et les données provenant de bases données,
j. une interface (19) ou un dispositif de saisie permettant à un utilisateur d'entrer des données, telles que par exemple les temps d'irradiation, la dose d'irradiation,
**caractérisé en ce que**
k. le système d'irradiation est conçu pour stabiliser biomécaniquement la cornée d'un oeil dans laquelle est introduit un photosensibilisant, tel que la riboflavine,
l. les données entrées dans l'interface (19) se référant à la stabilisation biomécanique de la cornée à l'aide du photosensibilisant, tel que la riboflavine, et
m. la commande (7) étant conçue pour commander ou régler ladite au moins une source de rayonnement (1) pour fournir un rayonnement conformément aux données entrées, par rapport à la stabilisation biomécanique de la cornée à l'aide du photosensibilisant,
n. ledit au moins un obturateur (12) pouvant être commandé pour ajuster la zone d'irradiation (13).

2. Système d'irradiation selon la revendication 1, comprenant au moins un élément (20) diffractif ou holographique dans la trajectoire du faisceau.

3. Système d'irradiation selon la revendication 1, comprenant au moins un modulateur de lumière placé dans la trajectoire du faisceau pour obtenir une répartition de lumière prédéterminée.

4. Système d'irradiation selon la revendication 1, comprenant des éléments optiques déplaçables situés dans la trajectoire du faisceau.

5. Système d'irradiation selon la revendication 1, ledit obturateur (12) pouvant être commandé électroniquement et se trouvant dans la trajectoire du faisceau.

6. Système d'irradiation selon la revendication 1, comprenant des moyens de mesure (9) pour le diagnostic en temps réel, en particulier pour la tomographie par cohérence optique ou pour la mesure optique de la longueur de l'oeil.

7. Système d'irradiation selon la revendication 1, comprenant au moins un des moyens de mesure suivants : la topographie de la cornée, le diagnostic de front d'ondes, la caméra de Scheimpflug, le système vidéo, le système de caméra, le microscope, le spectromètre, l'eye-tracker, le pachymètre, ou la mesure de l'épaisseur de l'épithélium, la mesure de la distance par rapport à l'oeil.

8. Système d'irradiation selon la revendication 1, comprenant un diviseur de faisceau (3) servant à combiner un rayonnement primaire provenant d'une première source de rayonnement (1) avec un rayonnement secondaire ayant une autre longueur d'onde que le rayonnement primaire, le rayonnement secondaire étant en particulier un rayonnement UV pour photo-ablation, un rayonnement pour excitation de la fluorescence du tissu, un rayonnement pour excitation thermique du tissu, ou un faisceau de visée ou d'un faisceau de fixation.

9. Système d'irradiation selon l'une des revendications précédentes, comprenant des moyens (25) pour ajuster et centrer le rayonnement.

10. Système d'irradiation selon l'une des revendications précédentes, comprenant un adaptateur (26) pour aligner le système d'irradiation et former le tissu à irradier.

11. Système d'irradiation selon l'une des revendications précédentes, comprenant un système de calibrage externe (31).

12. Système d'irradiation selon l'une des revendications précédentes, la commande (7) étant conçue pour commander ou régler dans le temps ladite au moins une source de rayonnement (1), conformément à la dose de radiation entrée par le biais de l'interface (19).
